# EUROPEAN PATENT APPLICATION

(11) **EP 4 378 467 A1**
(43) Date of publication of application: **05.06.2024**
(21) Application number: 21951959.2
(22) Date of filing: 24.12.2021
(51) Int. Cl.: A61K 33/06, A61P 1/10

(54) **MAGNESIUM OXIDE-CONTAINING PHARMACEUTICAL COMPOSITION FOR TREATING PEDIATRIC CHRONIC FUNCTIONAL CONSTIPATION**

(30) Priority: 30.07.2021 JP 2021126021
(71) Applicant: SETOLAS Holdings, Inc., Takamatsu-shi, Kagawa 760-0026 (JP)
(72) Inventor: YOSHIMURA, Yuya, Kita-gun, Kagawa 761-0705 (JP); ONISHI, Hazuki, Kita-gun, Kagawa 761-0705 (JP); SHIBUTANI, Daisuke, Kita-gun, Kagawa 761-0705 (JP)
(74) Representative: Vossius & Partner Patentanwälte Rechtsanwälte mbB
(86) International application number: PCT/JP2021/048377
(87) International publication number: WO 2023/007766

(57) **Abstract**

Provided is a pharmaceutical composition with which an adequate constipation treatment success rate is achieved in pediatric constipation patients aged 1 to 5. The pharmaceutical composition is for treating constipation and contains magnesium oxide as an active ingredient, the pharmaceutical composition being characterized in being orally administered twice a day to pediatric patients aged 1 to 5 and having a body weight of 7.5 kg or above, so that the dosage of magnesium oxide administered per day is 25 to 45 mg/kg.

## Description

### FIELD

The present invention relates to a pharmaceutical composition containing magnesium oxide as an active ingredient for treating pediatric chronic functional constipation.

### BACKGROUND

Pediatric chronic functional constipation is deemed as a disease that "occurs frequently and does not always have a favorable prognosis if not treated early and appropriately." Because the persistence of chronic functional constipation is accompanied by pain and distress, children withstand defecation to avoid pain and distress during defecation. This leads to the formation of hard stools and the loss of defecation desire, which worsen the conditions. It is therefore desired to release chronic functional constipation as quickly as possible.

Weaning, toilet training, and school ages are the most common periods and triggers for the onset of constipation in children, with the peak onset during toilet training at 2 to 4 years of age. It has been pointed out that repeated experience of unpleasant defecation during this period may lead to avoidance of defecation, whether consciously or unconsciously.

According to the Guidelines for the Diagnosis and Treatment of Pediatric Chronic Functional Constipation, edited by the Japanese Society for Pediatric Gastroenterology, Hepatology and Nutrition and the Japanese Society for Pediatric Neurogastroenterology, more than 40% of children diagnosed with constipation at age 4 or younger still have constipation symptoms at school age despite treatment with stool mass removal, laxatives and dietary fiber intake.

Pediatric chronic functional constipation has a significantly poorer prognosis when the age of the first visit is 2 years or older, and about 25% of children with constipation who present at childhood age 5 years or older will transition to adult constipation. Further factors contributing to poor prognosis include a longer time from onset to the first visit, and less frequent defecation at the first visit.

According to the aforementioned guidelines, drugs for chronic functional constipation in children with pediatric indications in Japan include maltz extract, lactulose, sodium picosulfate, bisacodyl, and glycerin. Of these, maltz extract, lactulose, and sodium picosulfate can be administered orally. Polyethylene glycol is known as a drug for oral administration outside Japan, and drugs containing polyethylene glycol have recently been approved in Japan (Non-Patent Literature 1).

In Japan, the general rule in the pharmacotherapy of chronic functional constipation in children is to start treatment with an osmotic laxative. According to this rule, among lactulose, sodium picosulfate, and polyethylene glycol, which have been approved as oral drugs for medical use, lactulose and polyethylene glycol may be selected. Available dosage forms for these drugs are syrup and powder for lactulose and oral liquid powder for polyethylene glycol. Thus, only lactulose powder exists as a prescription drug that can be administered orally as a solid dosage form.

The dosage and regimen approved for lactulose powder is oral administration of 0.33 to 1.30 g/kg per day in three divided doses. This means that the dosage for a child aged 1 year or older and weighing 10 kg or more is 1.1 to 4.3 g/day. This high dosage makes it more difficult for younger children to take the drug, while it is not easy to adjust the dosage according to their symptoms.

On the other hand, magnesium oxide powder is the most frequently used treatment for constipation in Japan. Although there is little evidence for this drug among various constipation medications, its efficacy and safety are considered clear when administered in appropriate doses. Magnesium oxide powder is listed among the most frequently used laxatives in the Guidelines for the Diagnosis and Treatment of Pediatric Chronic Functional Constipation.

Magnesium oxide is available for both medical and general use, and is most frequently used mainly for adults, especially for the elderly. While magnesium oxide for medical use is not indicated for use in children, magnesium oxide for general use can be indicated for children as young as 5 years old, and is taken orally at a dosage of from 0.33 to 0.66 g once a day before bedtime. When magnesium oxide for medical use is prescribed for constipation, adults should take 2 g of magnesium oxide equivalent per day orally in three divided doses before or after meals, or once before bedtime. On the other hand, the dosage and regimen for pediatric use has not been approved.

A report of a comparison study of the efficacy of approved lactulose versus unapproved magnesium oxide (Non-Patent Literature 2) discloses success rates in the treatment of constipation for children under 15 years of age diagnosed with chronic constipation, particularly those with a median age of 2 years and 11 months being the subject of analysis. The report states that the success rate for treatment of constipation was significantly higher in the group administered with unapproved magnesium oxide, 41% (20 of 49 cases), than in the lactulose group, 18% (9 of 50 cases). However, neither is deemed to provide adequate treatment for pediatric chronic functional constipation.

The dosage of magnesium oxide has not been established for infants 1 year or older. According to the aforementioned report of comparison study on the efficacy of magnesium oxide with lactulose (Non-Patent Literature 2), magnesium oxide was administered at a dose of 50.0 mg/kg per day. The number of doses for infants is also unknown, but is usually the same as for adults. According to the aforementioned report (Non-Patent Literature 2), the dosage was divided into two doses per day like for adults. When the target patients are children between 1 and 5 years of age, the drug is rarely administered in a form that is difficult to take, and is generally prescribed in a powder or liquid form (see Non-Patent Literature 3). In the aforementioned report (Non-Patent Literature 2), magnesium oxide was administered in the form of powder.

On the other hand, hypermagnesemia has been reported in patients administered with magnesium oxide for medical use, in which the oral administration of magnesium oxide caused abnormally high serum magnesium levels and led to death in severe cases, resulting in a warning given for its use. In general, renal dysfunction is indicated as a reason for abnormally elevated serum magnesium levels. In children with normal renal function, elevations in serum magnesium levels are minor and not clinically problematic. However, because children with chronic functional constipation require long-term treatment (Non-Patent Literature 4), attention must be paid to the dosage of magnesium oxide powder. Hence, the treatment provided by magnesium oxide should be effective in the short term.

To prevent abnormally high serum magnesium levels, it is recommended to reduce the dosage of magnesium oxide powder. However, the lower the single dose is, the more difficult the resulting powder is to handle. Furthermore, the duration of treatment for children with chronic functional constipation is prolonged due to significantly reduced therapeutic efficacy. As a result, drug therapy must be continued for at least several months in order to improve the pain and distress during defecation, dyspareunia, and irregular defecation habits that cause constipation (Non-Patent Literature 5).

Although a higher dosage of magnesium oxide powder is suggested to enjoy a therapeutic effect, it is also likely to cause evasion of medication and abnormally high serum magnesium levels due to the increased dosage, which may result in the risk of diarrhea.

It has been shown that tablets are not taken at the age of 1 to 2 years, and that powder are also problematic to take (Non-Patent Literature 6). Therefore, in the treatment of pediatric constipation patients between the ages of 1 and 5 years, if an appropriate formulation and its dosage can be established that allow for appropriate continuation of medication, it will be possible to maximize the therapeutic effect without concern for hypermagnesemia. As a result, children with constipation disorder are relieved of pain and distress during bowel movements as quickly as possible, and early release enables a good prognosis and prevents the transition to constipation in adults.

The present applicant has conducted various studies on magnesium oxide preparations for the treatment of chronic functional constipation. For example, the applicant has developed, as a magnesium oxide preparation with high disintegrating property to improve the compliance, tablets producing finer water-suspended particles, which are produced by adjusting the type of disintegrant, blending ratio, and manufacturing method (Patent Literature 1); tablets with improved tableting and disintegration properties, prepared by adjusting the average secondary particle diameter of magnesium oxide particles and the type and amount of binding agent and disintegrant (Patent Literature 2); and fine granules with improved solubility and texture, prepared by adjusting the average secondary particle diameter and apparent specific volume of magnesium oxide particles and granulating them with sugar alcohols and disintegrants (Patent Literature 3).

### CITATION LIST

### Patent Literature

[Patent Literature 1] WO2020/101016 A
[Patent Literature 2] JP4015485 A
[Patent Literature 3] WO2010/098417 A

### Non-Patent Literature

[Non-Patent Literature 1] Guidelines for the Diagnosis and Treatment of Pediatric Chronic Functional Constipation, 2013 edition
[Non-Patent Literature 2] The Journal of Ambulatory and General Pediatrics (Japanese journal), 19(2), 141-148, 2016
[Non-Patent Literature 3] Yakugaku Zasshi, 135(2), 2015, 245-247
[Non-Patent Literature 4] Japanese Journal of Pediatric Gastroenterology and Nutrition (Japanese journal), 29, Suppl, 2015, 126
[Non-Patent Literature 5] Journal of the Japanese Society of Pediatric Surgeons (Japanese journal), 49(3), 2013, 835
[Non-Patent Literature 6] Reflection Paper, Formulations of choice for the Pediatric Population, EMA, 28 July 2006

### SUMMARY

### Problem to be Solved

An objective of the present invention is to provide a pharmaceutical composition that achieves an adequate constipation treatment success rate for pediatric constipated patients between the ages of 1 and 5 years.

### Means to solve the Problem

The present inventors have diligently investigated appropriate dosage forms to explore the potential use of magnesium oxide formulation for the treatment of chronic functional constipation in pediatric patients. As a result, the present inventors have surprisingly found that lowering the daily dosage of magnesium oxide from the previously known 50.0 mg/kg (Non-Patent Literature 2) serves not only reduce the risk of side effects but also significantly improve the success rate of treatment. The present inventors have also examined the preferred dosage form and other factors and, as a result, have surprisingly found that, instead of the previously known magnesium oxide powder (Non-Patent Literature 2), a tablet or fine granule formulation, which is generally considered unsuitable for children, serves to improve the drug compliance rate and further improve the success rate of treatment. Based on these findings, the present inventors have completed the present invention.

One or more embodiments of the present invention relate to the following.
[Aspect 1] A pharmaceutical composition comprising magnesium oxide as an active ingredient for treating constipation, which is administered orally to a pediatric patient with a body weight of 7.5kg or more and an age of from 1 to 5 years old twice daily such that a daily dosage of magnesium oxide is from 25 to 45 mg/kg.
[Aspect 2] The pharmaceutical composition according to Aspect 1, which is in the form of tablets or fine granules.
[Aspect 3] The pharmaceutical composition according to Aspect 1 or 2, wherein the twice daily administration includes administrations after breakfast and after dinner.
[Aspect 4] The pharmaceutical composition according to any one of Aspects 1 to 3, wherein the daily dosage is administered as 2n units, which are divided into n units after breakfast and n units after dinner.
[Aspect 5] The pharmaceutical composition according to any one of Aspects 1 to 3, wherein the daily dosage is administered as 2n+1 units, which are divided into n units after breakfast and n+1 units after dinner.
[Aspect 6] The pharmaceutical composition according to any one of Aspects 1 to 5, wherein the constipation is chronic functional constipation.
[Aspect 7] Use of magnesium oxide in the preparation of a pharmaceutical composition according to any one of Aspects 1 to 6.
[Aspect 8] A method for treating constipation, comprising administering a pharmaceutical composition containing magnesium oxide as an active ingredient orally to a pediatric patient with a body weight of 7.5kg or more and an age of from 1 to 5 years old twice daily such that a daily dosage of magnesium oxide is from 25 to 45 mg/kg.
[Aspect 9] The method according to Aspect 8, wherein the pharmaceutical composition is in the form of tablets or fine granules.
[Aspect 10] The method according to Aspect 8 or 9, wherein the twice daily administration includes administrations after breakfast and after dinner.
[Aspect 11] The method according to any one of Aspects 8 to 10, wherein the daily dosage of the pharmaceutical composition is administered as 2n units, which are divided into n units after breakfast and n units after dinner.
[Aspect 12] The method according to any one of Aspects 8 to 11, wherein the daily dosage of the pharmaceutical composition is administered as 2n+1 units, which are divided into n units after breakfast and n+1 units after dinner.
[Aspect 13] The method according to any one of Aspects 8 to 12, wherein the constipation is chronic functional constipation.

### Effects of the Invention

The present invention provides a pharmaceutical composition that has excellent therapeutic effects and drivability and achieves an adequate constipation treatment success rate for pediatric constipated patients between the ages of 1 and 5 years.

### EMBODIMENTS

The present invention is described hereinafter in detail with reference to specific embodiments thereof. However, the present invention is not limited to the following embodiments and can be carried out in any embodiment that does not deviate from the gist according to the present invention. The various embodiments of the present invention described below are not in any way alternative or exclusive, and any two or more embodiments may be combined as appropriate, unless they result in apparent logical contradiction. Any combination of two or more of these embodiments as appropriate shall naturally be included in the scope of the present invention. When multiple upper limits and/or multiple lower limits are indicated for a numerical range, all numerical ranges obtained by combining any of the upper limits with any of the lower limits shall be included in the scope of the present invention.

### [Pharmaceutical composition]

An embodiment of the present invention provides a pharmaceutical composition containing magnesium oxide as an active ingredient for treating pediatric patients with constipation. According to an embodiment, the constipation to be treated by the pharmaceutical composition of the invention may preferably be chronic constipation, more preferably chronic functional constipation. As mentioned above, magnesium oxide formulations have not been approved for use in the treatment of children who have developed chronic constipation. On the other hand, magnesium oxidepowder are clinically prescribed as off-label use in some cases at the discretion of physicians. However, it is assumed that none of the drug manufacturers chose to make an effort to carry out clinical trials for obtaining an indication for magnesium oxide tablets from the standpoint of cost-effectiveness. Only the present inventors have arrived at the findings of the present invention, which provide various preferred embodiments based on the study of specific embodiments of magnesium oxide for use in the treatment of pediatric chronic functional constipation. These findings are of great importance in the treatment of pediatric patients with constipation, preferably pediatric patients with chronic functional constipation.

The pharmaceutical composition of the present invention is administered to a pediatric patient with a body weight of 7.5kg or more and an age of 1 to 5 years old such that the daily dosage of magnesium oxide is 25 to 45 mg/kg. Non-patent literature 2 is deemed to be virtually the only known report on the efficacy of magnesium oxide administration to pediatric patients with chronic functional constipation. Non-patent literature 2 shows that the treatment success rate for pediatric patients with chronic functional constipation was 41% when magnesium oxide powder was administered at a daily dosage of 50 mg/kg body weight per day. Usually, increased daily doses are attempted to increase treatment success rates. On the contrary, the present inventors have found that a significantly lower daily dosage (from 50 mg/kg body weight per day to 25-45 mg/kg body weight per day) not only reduces the risk of side effects, but also surprisingly significantly improves the treatment success rate even at an early stage. The finding of this effect is a surprising finding that far exceeds the level that a person skilled in the art can predict based on technical common knowledge.

According to an embodiment, the pharmaceutical composition of the present invention may preferably be in the form of tablets or fine granules containing magnesium oxide. Tablets or fine granules are not the treatment of choice for children aged 1 to 5 years since they are difficult to take. Instead, a powder or liquid formulation is usually prescribed (Non-Patent Literature 6). Magnesium oxide powder is also used in Non-Patent Literature 2, which is the only known report mentioned above. However, in addition to the reduction in daily dosage mentioned above, the present inventors dare to choose tablets or fine granules as the dosage form in order to improve the compliance rate. This allows for continuous medication compliance and, consequently, a high treatment success rate, even with short-term administration. Thus, this finding that tablets and fine granules can achieve the same or more compliance rates compared with conventional dispersions and liquids is a surprising finding that far exceeds the level that a person skilled in the art can predict based on technical common knowledge. Thus, the present invention provides a pharmaceutical composition with excellent therapeutic effects and dosing properties.

As mentioned above, the present applicant has conducted various studies on magnesium oxide formulations for treating chronic functional constipation, and has developed, as magnesium oxide formulations with high disintegrity for improving the ease of administration, tablets containing 100 mg per tablet (Patent Literature 1), tablets containing 200 mg per tablet (Patent Literature 2), and fine granules containing 83% per gram (Patent Literature 3). The knowledge of these tablets and fine granules makes it possible to produce tablets and fine granules that are easy for children aged 1-5 years to take. For children who have difficulty swallowing due to illness, tablets can be disintegrated and suspended in water, and the dispersion can be administered through a feeding tube. Examples of such tablets and granules will be described later.

According to an embodiment, the pharmaceutical composition of the present invention is divided and orally administered twice daily. According to an embodiment, the twice daily administration may preferably include administration after breakfast administration after dinner. According to an embodiment, the daily dosage of the pharmaceutical composition of the present invention may preferably be divided into 2n units, and n units may preferably be administered after breakfast and n units after dinner. According to an embodiment, the daily dosage of the pharmaceutical composition of the present invention may preferably be divided into 2n+1 units, and n units may preferably be administered after breakfast and n+1 units after dinner. Thus, when the number of dosage units per day is an odd number (2n+1), distributing more dosage units after dinner may have the advantage that the medicinal effect of magnesium oxide, whose onset time is 6 to 8 hours later, can be obtained after waking up, resulting in more appropriate defecation habits. It is said that as defecation habits, bowel movements may preferably occur in the morning.

### [pediatric chronic functional constipation]

As mentioned above, the therapeutic subject of the pharmaceutical composition of the present invention is constipation in pediatric patients, with chronic functional constipation being a particularly preferred therapeutic target. Pediatric chronic functional constipation is constipation or its symptoms that are manifested in children as conditions of stool retention or difficulty in passing stools, and is not organic constipation but requires medical treatment or therapy. The criteria internationally used to diagnose pediatric chronic functional constipation are the ROME IV criteria, which are the internationally used diagnostic criteria for chronic functional constipation. According to these criteria, chronic functional constipation is suspected when the following criteria are met.

### <Infants/young children>

For children younger than 4 years of age (infants or young children) or young adolescents, at least two of the following must be met within a minimum of one month.
1: Defecation twice per week or less
2: History of excessive stool retention
3: History of painful or hard bowel movements
4: History of large stools
5: Presence of large stool masses in the rectum

For children who have already received toilet training, the following also must be met.
6: Fecal incontinence at least once a week after learning toilet skills
7: History of stools large enough to clog the toilet

### <School children/young adolescents>

For children 4 years of age and older (school children) or young adolescents, at least two or more of the following symptoms must be met at least once per week during a minimum of one month, provided that the diagnostic criteria for irritable bowel syndrome are not met.
1: Defecate in the toilet twice a week or less
2: Fecal incontinence at least once a week
3: History of posture for holding stools or excessive spontaneous stool retention
4: History of painful or hard bowel movements
5: Presence of large stool masses in the rectum
6: History of stools large enough to clog the toilet
7: Symptoms that cannot be explained by other medical conditions after proper evaluation

The evaluation of stool condition when determining chronic functional constipation is performed according to the Bristol Stool Scale, which is a scale used to evaluate the condition of stools. According to the Bristol Stool Scale, stools are classified into the following seven levels based on their shapes and hardness.
1: Separate hard lump stools (rabbit-feces shaped stools)
2: Hard stools (sausage-shaped hard stools)
3: Slightly hard stools (sausage-shaped stools with cracks on their surfaces)
4: Normal stool (soft, half-solid stool with a smooth surface)
5: Slightly soft stools (wrinkled, soft, half-solid stools)
6: Muddy stools (irregularly shaped small pieces or muddy stools)
7: Watery stools (liquid stools without solids)

The diagnosis of constipation is made by asking each of the items in the ROME IV criteria and confirming whether or not the patient has constipation.

The goal of the treatment of pediatric chronic functional constipation is to reach or return to a non-constipated state and maintain it. The diagnosis may preferably be based on ROME IV criteria.

The effectiveness of the treatment is determined to be ineffective when the affected child and her/his fosterer understand the pathophysiology of constipation, the desired diet and defecation situation, and when a constipation-free state cannot be reached or maintained through drug treatment.

### [Composition and production method of formulations]

The dosage form of the pharmaceutical composition of the present invention is not limited, but as mentioned above, tablets and fine granules may be preferred. As mentioned above, the applicant has developed, as a magnesium oxide preparation with high disintegrating property to improve the compliance, tablets producing finer water-suspended particles, which are produced by adjusting the type of disintegrant, blending ratio, and manufacturing method (Patent Literature 1); tablets with improved tableting and disintegration properties, prepared by adjusting the average secondary particle diameter of magnesium oxide particles and the type and amount of binding agent and disintegrant (Patent Literature 2); and fine granules with improved solubility and texture, prepared by adjusting the average secondary particle diameter and apparent specific volume of magnesium oxide particles and granulating them with sugar alcohols and disintegrants (Patent Literature 3). Using the findings of these tablets and fine granules makes it possible to create tablets and fine granules that are easy for children aged 1-5 years to take.

The following is a description of the various dosage forms of the pharmaceutical compositions of the present invention, mainly in the case of tablets and fine granules. However, the pharmaceutical compositions of the present invention are not limited to these specific dosage forms. In the following descriptions, as appropriate, the pharmaceutical compositions of the present invention formulated into any dosage forms are collectively referred to as "the formulation of the present invention," and those formulated into tablets or fine granules, among others, may be referred to individually as "the present invention tablets" and "the present invention fine granules," respectively.

### *Magnesium oxide:

Magnesium oxide particles to be contained in the formulation of the present invention may have, although is not limited to, an elemental composition represented by, e.g., compositional formula (1) below. It may also preferably be, although is not limited to, magnesium oxide specified in the Japanese Pharmacopoeia.

(Mg²⁺_{1-X}Zn²⁺_{X})O (1)

In the above formula, X represents a number of from 0 to 0.02. Magnesium oxide particles with X greater than 0 are not mixtures of magnesium oxide and zinc, but rather have the same crystal structure as that of magnesium oxide, in which zinc atoms are stuck in the crystal structure of magnesium oxide. The magnesium oxide particles show the same diffraction pattern as magnesium oxide according to the powder X-ray diffraction method. X may be a number of 0 or more, or 0.001 or more, or 0.005 or more, and 0.02 or less, or 0.015 or less, or 0.01 or less. If the value of X is too high, the total amount of Zn, including Zn taken from food, may exceed the required amount of Zn as an essential mineral.

The volume-based 50% particle diameter (D₅₀) of magnesium oxide particles to be contained in the formulation of the present invention, as measured by laser diffraction scattering, may be, although is not limited to, usually 0.1 µm or more, or 0.5 µm or more, or 1 µm or more, and usually 25 µm or less, or 20 µm or less, or 18 µm or less, or 15 µm or less, or 10 µm or less. Especially in the case of fine granules, if the particle size of magnesium oxide is too large, the disintegration time may be so long that the particles may not disperse quickly in the oral cavity.

The volume-based 50% particle diameter (D₅₀) of magnesium oxide is measured by the following method. 0.7 g of magnesium oxide and 70 mL of 0.2% sodium hexametaphosphate solution are put into a beaker and subjected to dispersion processing using an ultrasonic homogenizer (US-300, Nippon Seiki). The resulting dispersion is then subjected to measurement using a laser diffraction scattering particle size analyzer (Nikkiso, Microtrac) to thereby determine the volume-based 50% particle diameter (D₅₀) of the aforementioned magnesium oxide.

The apparent specific volume of magnesium oxide particles to be contained in the formulation of the present invention is not limited, but especially in the case of fine granules, it may be usually 3 mL/g or more, or 4 mL/g or more, and usually 20 mL/g or less, or 15 mL/g or less. Especially in the case of fine granules, if the apparent specific volume of magnesium oxide particles is too small, elution may be poor. On the other hand, if the apparent specific volume of magnesium oxide particles is too large, the bulk of the particles may increase and make granulation difficult.

Magnesium oxide particles to be contained in the formulation of the present invention may be produced by a method including, although not limited to, calcinating hydroxide magnesium particles. The specific procedure may be, although is not limited to, as follows. Magnesium hydroxide particles as raw material may be produced by a method including, although not limited to, precipitating magnesium ions in seawater or bitter water as magnesium hydroxide using an alkaline source. Examples of alkali sources include, but are not limited to, calcium hydroxide, caustic soda, potassium hydroxide, lithium hydroxide, and ammonia water, among which caustic soda or calcium hydroxide are particularly preferred. The precipitated magnesium hydroxide is then subjected to calcination after heat treatment at, e.g., 100°C to 120°C. The calcination conditions are not limited, but may be carried out, for example, usually at 500°C or more, or 600°C or more, and usually at 1,000°C or less, especially 900°C or less, for usually 0.1 to 10 hours. If the calcination temperature is too high or the calcination time is too long, the magnesium oxide particles may become so hard that it may disintegrate poorly.

Magnesium oxide particles in which zinc (Zn) is solidly dissolved in magnesium oxide may be produced by a method including, although not limited to, preparing an aqueous solution containing magnesium and zinc (Zn) cations, adding an alkaline substance of approximately equivalent or less to the total equivalent of these cations to the solution, and causing a reaction in the solution under agitation. If necessary, the reactants may be further hydrothermally treated in an autoclave at 100°C to 200°C. Then, like the production of magnesium oxide, the reaction product may be washed, dehydrated, dried, and calcined, and also may be subjected to other customary treatments as appropriate, such as crushing or classification. Examples of sources for magnesium ions may include, although are not limited to, magnesium nitrate and magnesium chloride. Examples of sources for zinc (Zn) ions may include, although are not limited to, zinc nitrate and zinc chloride. Examples of alkaline substances may include, although are not limited to, sodium hydroxide.

### *Binder:

The formulation of the present invention may contain a binder. Examples of binders may include, although are not limited to, crystal cellulose, carboxyl methyl cellulose sodium, low-substituted hydroxy propyl cellulose, starch (e.g., corn starch), and sugar alcohol. Any one of these may be used alone, or any two or more of these may be used in any combination and at any ratios. The timing of addition is also not limited. The content of the binder in the formulation of the present invention is also not limited, but in the case of tablets, it may be typically 1 mass % or more, or 3 mass % or more, and typically 15 mass % or less, or 13 mass % or less.

### *Disintegrant:

The formulation of the present invention may contain a disintegrant. Examples of disintegrants may include, although are not limited to, starch, croscarmellose sodium, crospovidone low-substituted hydroxy propyl cellulose, carmellose calcium, carmellose, carboxyl starch sodium, and insoluble polyvinyl pyrrolidone. Any one of these may be used alone, or any two or more of these may be used in any combination and at any ratios. The content of the disintegrant in the formulation of the present invention is also not limited, but it may be typically 0.5 mass % or more, or 1 mass % or more, and typically 10 mass % or less, or 7 mass % or less.

The volume-based 50% particle diameter (D₅₀) of the disintegrant contained in the formulation of the present invention, as measured by laser diffraction scattering, is also not limited, but it may be typically 0.1 µm or more, or 0.5 µm or more, or 1 µm or more, and typically 150 µm or less, or 100 µm or less, or 25 µm or less, or 15 µm or less, or 10 µm or less.

The volume-based 50% particle diameter (D₅₀) of the disintegrant is measured by a laser diffraction scattering particle size analyzer (LMS-2000e by Seishin Enterprise Co., Ltd.). Measurement for particle size distribution is carried out by a dry method without using dispersants to determine the volume-based 50% particle diameter (D₅₀).

### *Lubricant:

The formulation of the present invention may contain, especially in the case of tablets, a lubricant. Examples of lubricants may include, although are not limited to, stearic acid and its salts (Na, Mg, Ca salts). Preferred among these include calcium stearate. Any one of these may be used alone, or any two or more of these may be used in any combination and at any ratios. The content of the lubricant in the tablets of the present invention is also not limited, but it may be typically 0.2 mass % or more, or 0.5 mass % or more, or 0.7 mass % or more, and typically 3 mass % or less, or 2 mass % or less, or 1.5 mass % or less.

### *Sugar alcohol:

The formulation of the present invention may contain, especially in the case of fine granules, a sugar alcohol. Examples of sugar alcohols may include, although are not limited to, xylitol, erythritol, sorbitol, and mannitol. Preferred among these include mannitol. Any one of these may be used alone, or any two or more of these may be used in any combination and at any ratios. The content of the sugar alcohol in the fine granules of the present invention is also not limited, but it may be typically 2 mass % or more, or 5 mass % or more, or 6 mass % or more, and typically 15 mass % or less, or 10 mass % or less, or 9 mass % or less.

### *Other ingredients:

The formulation of the present invention may contain other additional ingredients, depending on its dosage form. Any one of these additional ingredients may be used alone, or any two or more of these may be used in any combination and at any ratios. Examples include sweeteners, flavoring powders, and taste masking agents. Examples of sweeteners may include, although are not limited to, aspartame, acesulfame potassium, and sucralose. Examples of flavoring powders may include, although are not limited to, peppermints, L-menthol, orange powder, and strawberry essence. Examples of corrigents may include, although are not limited to, white sugar, mannitol, and sorbitol. The content of the additional ingredients in the formulation of the present invention is also not limited, but it may be typically 0.05 mass % or more, or 0.1 mass % or more, or 0.2 mass % or more, and typically 2 mass % or less, or 1 mass % or less, or 0.5 mass % or less.

### *Production methods for tablets and fine granules:

The method for producing the formulation of the present invention is not limited, and an appropriate method for the dosage form may be selected for the production. The following description will be made mainly taking the case of tablets and fine granules as examples, but the production methods for the formulation of the present invention are not limited to these examples.

When the formulation of the present invention is to be made into tablets, a raw material mixture for tableting is first prepared. The raw material mixture can be prepared by mixing an active pharmaceutical ingredient, i.e., magnesium oxide particles, along with a part or all of the aforementioned binding agent and disintegrant, and optionally with other additional ingredients as appropriate. The mixing may be carried out using a mixer such as a container type, V-type, or W-type. The raw material mixture is then granulated into granular particles. This granulation can be carried out using, e.g., a dry granulator. Examples of dry granulators include roll forming dry granulators. In such cases, the roll pressure may be typically 3 MPa or more, or 4 MPa or more, and typically 12 MPa or less, or 8 MPa or less. The granulated sheet-like compact is then pulverized to obtain granular particles. The pulverization can be carried out using, e.g., an oscillator-type mill. The opening size of the screen for the oscillator may be, although are not limited to, typically 0.7 mm or more, or 0.8 mm or more, and typically 1.2 mm or less, or 1.0 mm or less. The size of the granular particles is not limited, but may be such that the average particle diameter is 0.25 to 0.4 mm, the apparent density is 0.5 to 0.7 g/mL, and the angle of repose is 35 to 43°.

The granular particles can be mixed with the lubricant as needed, optionally with the remainder of the aforementioned binders and disintegrants, and optionally with the additional ingredients, and the mixture is then tableted to form a tablet. The tableting pressure per tablet may be typically 5 kN or more, or 6 kN or more, and typically 12 kN or less, or 10 kN or less, but any tableting pressure may be selected according to the shape of the tableting punch and the mass of each tablet. The shape of the tableting punch may be a round plane, a round-cornered round plane, a sharp-cornered flat plane, or round-cornered flat plane. The diameter of the tablet is also not limited, but it may be typically 4 mm or more, or 5 mm or more, and typically 12 mm or less, or 10 mm or less, or 8 mm or less. The thickness of the tablet is also not limited, but it may be typically 2 mm or more, or 2.5 mm or more, and typically 6 mm or less, or 5 mm or less, or 4.5 mm or less. The mass per tablet is also not limited, but it may be typically 50 mg or more, or 70 mg or more, or 90 mg or more, and typically 300 mg or less, or 280 mg or less, or 250 mg or less. When the size and mass of each tablet is adjusted to within the above ranges, the resulting tablets may be easy for children to swallow.

On the other hand, the formulation of the present invention is made into fine granules, a raw material mixture for granulation is first prepared. The raw material mixture can be prepared by mixing an active pharmaceutical ingredient, i.e., magnesium oxide particles, along with the binder and the disintegrant mentioned above, and optionally with the sugar alcohol and the additional ingredients mentioned above as appropriate. The mixing may be carried out using a mixer such as a container type, V-type, or W-type. The raw material mixture is then granulated into granular particles. This granulation can be carried out using, e.g., a dry granulator. Examples of dry granulators include roll forming dry granulators. In such cases, the roll pressure may be typically 3 MPa or more, or 4 MPa or more, and typically 12 MPa or less, or 8 MPa or less. The granulated sheet-like compact is then pulverized to obtain granular particles. The pulverization can be carried out using, e.g., an oscillator-type mill. The opening size of the screen for the oscillator may be, although are not limited to, typically 0.7 mm or more, or 0.8 mm or more, and typically 1.2 mm or less, or 1.0 mm or less. The size of the granular particles is not limited, but the average particle diameter may be 0.25 to 0.4 mm, the apparent density may be 0.5 to 0.7 g/mL, and the angle of repose may be 35 to 43°.

The granular particles are classified using, e.g., a vibrating sieve (e.g., 0.15 mm or 0.5 mm) to produce fine particles with an average particle diameter (X₅₀) of, e.g., from 0.2 mm to 0.4 mm or from 0.25 mm to 0.35 mm. The bulk density of the fine particles is also not limited, but it may be typically 0.4 g/mL or more, or 0.5 g/mL or more, and typically 0.7 g/mL or less, or 0.65 g/mL or less, or 0.6 g/mL or less. The particle size distribution of the fine particles is also not limited, but the ratio of particles with particle diameters of within the range of 355 µm or more but less than 500 µm may be typically 30 mass % or more, or 32 mass % or more, and typically 45 mass % or less, or 42 mass % or less, the ratio of particles with particle diameters of within the range of 180 µm or more but less than 355µm may be typically 40 mass % or more, and typically 50 mass % or less, or 49 mass % or less, and the ratio of particles with particle diameters of within the range of 150 µm or more but less than 180µm may be typically 10 mass % or more, and typically 28 mass % or more, or 27 mass % or more. The resulting fine particles may optionally be mixed with other ingredients in a container or mixer. Among other things, a small amount of peppermint cortone or L-menthol may be adsorbed on hydrous silicon dioxide as a flavoring powder and used, since it may improve the taste.

When the formulation of the present invention is made into tablets or fine granules, children who have difficulty swallowing due to illness may choose to have the formulation suspended in water and administered by tube using a feeding tube. For tube administration to pediatric patients, it is said that a 3Fr (1.0mm OD) or 4Fr (1.3mm OD) feeding tube may be preferred, depending on the child's weight. The average particle size produced when the formulation is suspended in water is not limited, but may be, for example, as follows. The volume-based 50% particle diameter (D₅₀) of the formulation of the present invention as measured by laser diffractometry may be 70 µm or less, or 60 µm or less, or 50 µm or less, and 20 µm or more, or 30 µm or more, or 40 µm or more. The volume-based 90% particle diameter (D₉₀) of the formulation of the present invention as measured by laser diffractometry may be 130 µm or less, or 120 µm or less, or 110 µm or less, and 50 µm or more, or 60 µm or more, or 70 µm or more. Adjusting the suspended particle size of the formulation within the above range makes it possible to achieve a higher dose rate for children who have difficulty swallowing, as they can be administered using a feeding tube.

When the formulation of the present invention is made into tablets or fine granules, the volume-based 50% particle size (D₅₀) and the volume-based 90% particle size (D₉₀) of the formulation after suspension in water are measured by the following method. 10 tablets of the formulation are put into a beaker with 40 mL of ion-exchanged water, and disintegrated and suspended using a glass rod. Measurement is carried out using a laser diffraction particle size analyzer (LMS-2000e from Seishin Enterprise Co., Ltd.) to determine the volume-based 50% particle size (D₅₀) and the volume-based 90% particle size (D₉₀) of the formulation suspended in water.

### *Others:

The composition and the production method of the formulation of the present invention have been described above, mainly taking tablets and fine granules as examples. Further details can be found, e.g., in the descriptions of Patent Literatures 1 to 3 mentioned above. However, the formulation of the present invention is not limited to the above descriptions in these patent literature documents, but can be implemented by changing the conditions as appropriate.

Various aspects of the present invention are not limited to the pharmaceutical compositions of the present invention and their dosage forms described above, but also encompass various other inventions that may be understood by a person skilled in the art based on these descriptions. For example, an aspect of the present invention relates to the use of magnesium oxide in the manufacture of the pharmaceutical composition of the present invention described above for treating constipation. Another aspect of the present invention relates to a method for treating constipation, including administering to a patient in need thereof a pharmaceutical composition of the present invention, which contains magnesium oxide. The following details of these aspects of the present invention are the same as those previously described in relation to the composition of the present invention: the details of the ingredients, dosage forms, physical properties, production methods, etc., of the pharmaceutical composition of the present invention; the shapes and physical properties, etc., of magnesium oxide to be used as the active pharmaceutical ingredient; the age, symptoms, weight, etc., of the patient to be treated; and the dosages and administration methods (dosages and regimens) of the pharmaceutical composition of the present invention to be administered to the patient.

### EXAMPLES

The present invention will be described in more detail in the following examples. However, these examples are only shown for convenience of explanation, and the present invention is not limited to these examples in any sense.

### [Example 1]

In this example, the efficacy of tablets containing 100 mg of magnesium oxide was examined in pediatric patients with chronic functional constipation aged 1 to 5 years.

Administered formulation: Magnesium oxide (100 mg tablets). Prepared in accordance with the descriptions on paragraphs [0019] to [0026] and Example 3 of Patent Literature 1.

### Patents as the subject for administration: 26 cases.

Regimen/dosage: The number of tablets to be administered daily and the initial dose were determined according to Table 1 below, based on the patient's body weight at the start of administration, to be approximately 40 mg/kg of magnesium oxide per day. When the daily dosage was 2n tablets, n tablets were administered after breakfast and n after dinner, while when the daily dosage was 2n + 1 tablets, n tablets were administered after breakfast and n + 1 after dinner. Specifically, the daily dosage was divided and orally administered as shown in Table 1 below.

**[Table 1]**

| Table 1. Correspondence between patient body weight and the numbers of tablets administered per day, after breakfast, and after dinner | | | |
|---|---|---|---|
| Body weight of patient | Number of tablets administered per day | Number of tablets administered after breakfast | Number of tablets administered after dinner |
| 7.5 to 10.0 kg | 3 | 1 | 2 |
| 10.0 to 12.5 kg | 4 | 2 | 2 |
| 12.5 to 15.0 kg | 5 | 2 | 3 |
| 15.0 to 17.5 kg | 6 | 3 | 3 |
| 17.5 to 20.0 kg | 7 | 3 | 4 |
| 20.0 to 22.5 kg | 8 | 4 | 4 |
| 22.5 to 25.0 kg | 9 | 4 | 5 |
| 25.0 to 27.5 kg | 10 | 5 | 5 |

Dose adjustment: Starting with the initial dose, the dose can be increased or decreased until achieving adequate defecation (i.e., until reaching a state where spontaneous defecation of solid stools occurs at least 3 times/week, there is no persistent separate lumps or hard stools, and there is no muddy or watery stool). For patients with fecal masses identified prior to administration, the fecal masses were removed before the administration was started.

Administration period: Administration was continued for 4 weeks.

Analysis method: The frequency of spontaneous defecation and the scores of stool status were collected using electronic or non-electronic patient diaries for the last week to evaluate the number of defecations per week and the scores for stool status as listed in the ROME IV criteria. 4 weeks later, blood samples were collected to measure the serum magnesium concentrations.

Evaluation method: Summary statistics were calculated for the frequency of spontaneous defecation and stool status scores (scores according to the Bristol Stool Scale) during the last week, the percentage of patients who did not meet the ROME IV criteria for the diagnosis of constipation during the last week, medication compliance, trends in serum magnesium levels, and the dose administered per body weight during the last week, and a statistical analysis was performed on the treatment success rate. In the absence of defecation for 2 days, the use of bisacodyl suppositories or glycerin enemas (rescue medication) was allowed, and to distinguish spontaneous defecation, 24 hours after the use of rescue medication was not counted as spontaneous defecation.

Table 2 shows the patient background of the magnesium oxide (100 mg tablet) group.

**[Table 2]**

| Table 2. Patient background of the group receiving magnesium oxide administration (100 mg tablet) | | |
|---|---|---|
| Number of patients (cases) | | 26 |
| Sex | Male (cases) | 9 |
| | Female (cases) | 17 |
| Age | 1 year old (cases) | 6 |
| | 2 to 3 years old (cases) | 11 |
| | 4 to 5 years old (cases) | 9 |
| Average frequency of spontaneous defecation (times/week)* | | 3.4 |
| Stool status score per week according to the Bristol Stool Scale (median)* | | 2.9 |
| Serum magnesium concentration (mg/dL) | | 2.3 |

| | | |
|---|---|---|
| * To confirm defecation frequency and stool status scores due to chronic functional constipation, defecation status prior to the start of treatment was investigated in patients who consented. | | |

The frequency of spontaneous defecation during the last week of continuous administration for 4 weeks was 5.0 ± 2.8 (95% confidence interval: 3.81-6.11), with a minimum value of 1 and a maximum value of 15.

Table 3 shows the frequency of spontaneous defecation in the last week of each age group.

**[Table 3]**

| Table 3. Number of spontaneous defecations in the final week of each age group | | | |
|---|---|---|---|
| | Average ± standard deviation (times) | Minimal value (times) | Maximum value (times) |
| 1 year old | 3.8±1.5 | 2 | 6 |
| 2 to 3 years old | 4.3±2.1 | 1 | 9 |
| 4 to 5 years old | 6.6±3.7 | 2 | 15 |

The median fecal status score (according to the Bristol fecal scale) for the final week of 4 weeks of continuous administration was 5.0, with a minimum score of 3 and a maximum score of 6.

Table 4 shows the stool status score for the last week of each age group.

**[Table 4]**

| Table 4. Stool status score for the last week of each age group | | | |
|---|---|---|---|
| | Median | Minimum | Maximum |
| 1 year old | 4.6 | 3 | 5.5 |
| 2 to 3 years old | 4.9 | 4 | 6 |
| 4 to 5 years old | 5.5 | 5 | 6 |

The ratio of patients who did not meet ROME IV criteria for the diagnosis of constipation in the final week of the 4-week continuous administration was 21 of 26 patients (81%).

The mean medication compliance rate during the 4-week continuous administration was 99%.

The serum magnesium concentration was 2.3mg/dL before the start of administration, 2.4 mg/dL after 2 weeks of continuous administration, and 2.4 mg/dL after 4 weeks of continuous administration. None of the patients had hypermagnesemia.

When administered continuously for 4 weeks, the weekly administration dosage in the final week was 32.97±7.4 mg/kg/day (maximum: 45.1 mg/kg/day; minimum: 17.1 mg/kg/day; 95% confidence interval: 30.11 to 35.83 mg/kg/day).

Table 5 shows the weekly administration dosage in the final week for each age group.

**[Table 5]**

| Table 5. Weekly administration dosage in the final week for each age group (mg/kg/day) | | | |
|---|---|---|---|
| | Average | Minimum | Maximum |
| 1 year old | 32.90 | 25.2 | 36.7 |
| 2 to 3 years old | 32.26 | 20.8 | 43.2 |
| 4 to 5 years old | 33.88 | 17.1 | 45.1 |

Patients who met the following criteria were defined as those who were successfully treated for constipation: the frequency of defecation was at least 3 times/week, the stool status score was from 2 to 7, and no symptoms selected from encopresis, abdominal pain, pain during defecation, and bleeding during defecation was observed. With these criteria, the percentage of patients treated successfully for constipation was 73% (19 of 26 patients).

The posterior distribution treatment success probability can be calculated from the ratio of successful treatment cases (19 of 26 cases were successful). The posterior distribution is a distribution calculated by adding data to a prior distribution, which is derived from the probability distribution in Bayesian statistics (beta distribution) and represents parameter uncertainty. Analysis using this posterior distribution is deemed to be a scientifically valid method of combining current data with a prior distribution based on available prior test data. The posterior distribution makes it possible to derive probabilities of outcomes that will be observed in the future. The probability that the posterior distribution treatment success probability was greater than 70% was calculated using the statistical analysis software R (R version 3.4.2).

Since 19 of the 26 cases were successfully treated cases, the prior distribution has 19 treatment successes and 7 failures. The prior distribution and likelihood were combined to obtain a posterior distribution with 20 treatment successes and 8 failures. From this posterior distribution, the probability that the posterior distribution treatment success probability was 0.7 or less was calculated to be 0.411. That is, the probability that the treatment success rate was 70% or more was 58.9%.

Likewise, after 2 weeks of continuous administration, the frequency of spontaneous defecation per week was 5.5 ± 3.3 (95% confidence interval: 4.20 to 6.88), and the median weekly stool status score (according to the Bristol Stool Scale) was 5.2. Patients who met the following criteria were defined as those who were successfully treated for constipation: the frequency of defecation was at least 3 times/week, the stool status score was from 2 to 7, and no symptoms selected from encopresis, abdominal pain, pain during defecation, and bleeding during defecation was observed. With these criteria, the percentage of patients treated successfully for constipation was 73% (19 cases of 26 cases). Since 19 of the 26 cases were successfully treated cases, the probability that the posterior distribution treatment success probability was 0.7 or less was calculated to be 0.411. That is, the probability that the treatment success rate was 70% or more was 58.9%. Thus, the treatment was confirmed to be effective from the early stage of treatment.

### [Example 2]

In this example, the subjects of study were pediatric chronic functional constipation patients aged 1 to 5 years who exhibited a defecation frequency of 4 times/week or more after receiving continuous administration of tablets containing magnesium oxide (100 mg tablets) for 8 weeks. The equivalence of these subjects was examined in an open-label, between-group study in which the subjects received administration of tablets (200 mg tablets) or fine granules (83% fine granules) containing magnesium oxide.

### Administered formulation:

*Magnesium oxide (100 mg tablets). Like Example 1, prepared in accordance with the descriptions on paragraphs [0019] to [0026] and Example 3 of Patent Literature 1.

*Magnesium oxide (200 mg tablets). Prepared in accordance with the descriptions on paragraphs [0024] to [0029] of Patent Literature 2.

*Magnesium oxide (83 % fine granules). Prepared in accordance with the descriptions on paragraph [0005] of Patent Literature 3.

### Patents as the subject for administration:

*Magnesium oxide (100 mg tablets): administered to 29 subjects
*Magnesium oxide (200 mg tablets): administered to 8 subjects
*Magnesium oxide (83% fine granules): administered to 21 subjects

Regimen/dosage: Like [Example 1], the number of tablets to be administered daily and the initial dose were determined according to Table 2 below, based on the patient's body weight at the start of administration, to be approximately 40 mg/kg of magnesium oxide per day. When the number of daily doses of 100 mg tablets after 8 weeks of continuous administration was 4 n tablets, 200 mg tablets were administered orally at 2 n tablets per day. When the number of daily doses of 100 mg tablets was not 4 n tablets, 83% fine granules were administered orally twice daily after breakfast and after dinner in the same dose (as magnesium oxide) as the 100 mg tablets.

Administration period: Both 200 mg tablets and 83% fine granules were administered continuously for 2 weeks.

Analysis method: For the final week, the frequency of spontaneous defecation and the scores for stool status were collected by electronic or non-electronic patient diaries to evaluate the frequency of spontaneous defecation and the scores for stool status per week as listed in the ROME IV criteria.

Evaluation method: Summary statistics were calculated for the change in the frequency of spontaneous defecation between the week before the switch to the administration of 100 mg tablets and the final week after the switch to the administration of 100 mg tablets, the stool status scores (according to the Bristol Stool Scale), the ratios of patients not meeting the ROME IV criteria, and the compliance rate, and statistical analyses were conducted for treatment success rates. Like [Example 1], in the absence of defecation for 2 days, the use of bisacodyl suppositories or glycerin enemas (rescue medication) was allowed, and to distinguish spontaneous defecation, 24 hours after the use of rescue medication was not counted as spontaneous defecation.

Table 6 shows the patient background for each dose group administered with 200 mg tablets or 83% fine granules.

**[Table 6]**

| Table 6. Patient background for each dose group | | | |
|---|---|---|---|
| | | 200 mg tablets Administration group | 83% fine granules Administration group |
| Number of patients (cases) | | 8 | 21 |
| Sex | Male (cases) | 3 | 10 |
| | Female (cases) | 5 | 11 |
| Age | 1 year old (cases) | 1 | 5 |
| | 2 to 3 years old (cases) | 3 | 9 |
| | 4 to 5 years old (cases) | 4 | 7 |
| Average frequency of spontaneous defecation per week before switching from 100 mg tablets (times/week)* | | 5.5 | 6.9 |
| Stool status scores according to the Bristol Stool Scale for 1 week before switching from 100 mg tablets (median)* | | 5.1 | 4.6 |

| | | | |
|---|---|---|---|
| *The defecation status before the switch was investigated in order to target patients who had achieved an efficacy of at least 4 times of defecation/week with the administration of tablets containing magnesium oxide (100 mg tablets). | | | |

The frequency of spontaneous defecation during the last week of the 2-week administration period after the switch was 5.5 ± 2.9 (95% confidence interval: 3.05-7.95) in the 200 mg tablet group and 5.8 ± 2.3 (95% confidence interval: 4.76-6.86) in the 83% fine granule group. The change from the frequency of spontaneous defecation during the week before the switch to 100 mg tablets was 0 ± 2.2 and -1.1 ± 2.2 times, respectively.

Table 7 shows the frequency of spontaneous defecation during the last week in each age group of each administration group.

**[Table 7]**

| Table 7. Frequency of spontaneous defecation during the last week in each age group of each administration group | | | | |
|---|---|---|---|---|
| | Administration group | Average ± standard deviation (times) | Minimum (times) | Maximum (times) |
| 1 year old | 200 mg tablets | 10 | 10 | 10 |
| | 83% fine granules | 4.4±3.0 | 0 | 8 |
| 2 to 3 years old | 200 mg tablets | 4.0±1.7 | 2 | 5 |
| | 83% fine granules | 6.2±1.8 | 4 | 9 |
| 4 to 5 years old | 200 mg tablets | 5.5±3.0 | 3 | 9 |
| | 83% fine granules | 6.3±2.3 | 4 | 11 |

The median stool status scores (according to the Bristol Fecal Scale) in the final week of continuous administration when the 2-week continued administration after the switch were 4.8 for the 200 mg tablet group and 4.3 for the 83% fine granule group.

Table 8 shows the stool status scores for the last week in each age group of each administration group.

**[Table 8]**

| Table 8. Stool status scores for the last week in each age group of each administration group | | | | |
|---|---|---|---|---|
| | Administration group | Median | Minimum | Maximum |
| 1 year old | 200 mg tablets | 3.0 | 3 | 3 |
| | 83% fine granules | 3.8 | 3 | 4 |
| 2 to 3 years old | 200 mg tablets | 4.7 | 4 | 5 |
| | 83% fine granules | 4.3 | 4 | 5 |
| 4 to 5 years old | 200 mg tablets | 5.3 | 4 | 6 |
| | 83% fine granules | 4.5 | 2 | 6 |

After 2 weeks of continuous administration, the mean compliance rate was 100% for the 200 mg tablets administration group and 99.8% for the 83% fine granules administration group.

Table 9 shows the change from the frequency of spontaneous defecation in the week before the switch to the frequency of spontaneous defecation in the final week of the 2-week continuous administration after the switch with 95% confidence intervals, which indicate that the equivalence margin of ±2 times is met.

**[Table 9]**

| Table 9. Change in spontaneous defecation frequency in each administration group | | | |
|---|---|---|---|
| Administration group | Change in spontaneous defecation frequency (times) | 95% confidence intervals | Judgment (variations are within ± times) |
| 200 mg tablets and 83% fine granules | -0.8±2.2 | -1.6 to 0.1 | Equivalent |

The equivalence margin is a range within which different therapeutic agents are considered equivalent in order to verify the equivalence of their efficacy. In Example 2, patients who had "defecation frequency of 4 times/week or more" after receiving 100 mg tablets were considered to be effective, and "defecation frequency of 2 times or less per week" indicates ongoing constipation in accordance with the ROME IV criteria. Therefore, the equivalence was evaluated with setting the equivalence margin at 2 times, in order to ensure that 4 times of defecation/week would not be less than 2 times/week.

The results of the equivalence evaluation showed that the frequency of defecation observed with each of the 100 mg and 200 mg tablets and the 83% fine granules demonstrated equivalent therapeutic efficacy. The 200 mg tablets and 83% fine granules could be taken continuously at the same doses as the 100 mg tablets, and the therapeutic effects thereof were as follows. With the 200 mg tablet administration group, the frequency of spontaneous defecation was 5.5 ± 2.9 times (95% confidence interval: 3.05-7.95), and the median stool status score (according to the Bristol Stool Scale) was 4.8. With the 83% fine granules, the frequency of spontaneous defecation was 5.8 ± 2.3 (95% confidence interval: 4.76-6.86), and the median stool status score (according to the Bristol Stool Scale) was 4.3. These results mean that pediatric chronic functional constipation from 1 to 5 years of age were successfully treated with tablets and fine granules.

Patients who met the following criteria were defined as those who were successfully treated for constipation: the frequency of defecation was at least 3 times/week, the stool status score was from 2 to 7, and no symptoms selected from encopresis, abdominal pain, pain during defecation, and bleeding during defecation was observed. With these criteria, the percentage of patients treated successfully for constipation was 88% (7 of 8 cases) for the 200 mg tablets administration group and 95% (20 of 21 cases) for the 83% fine granules administration group.

In the 200 mg tablets administration group, 7 of the 8 cases were treatment success cases, resulting in a prior distribution of 7 treatment successes and 1 failure. The prior distribution and likelihood were combined to obtain a posterior distribution with 8 treatment successes and 2 failures. From this posterior distribution, the probability that the posterior distribution treatment success probability was less than 0.7 was calculated to be 0.196. That is, the probability that the treatment success rate was greater than 70% was 80.4%.

In the 83% fine granules administration group, 20 of the 21 cases were treatment success cases, resulting in a prior distribution of 20 treatment successes and 1 failure. The prior distribution and likelihood were combined to obtain a posterior distribution with 21 treatment successes and 2 failures. From this posterior distribution, the probability that the posterior distribution treatment success probability was less than 0.7 was calculated to be 0.004. That is, the probability that the treatment success rate was greater than 70% was 99.6%.

### [Comparative Example 1]

In this comparison example, the results of a known report are indicated, in which the efficacy of magnesium oxide bulk powder was evaluated in 52 children under 15 years of age diagnosed with chronic constipation according to ROME III criteria (Non-Patent Literature 2).

Magnesium oxide: administered to 49 cases

Regimen/dosage: A daily dose of 50 mg/kg (maximum dose: 2000 mg/day) of magnesium oxide powder was orally administered in two divided doses.

Administration period: Administration was continued for 3 weeks.

Analysis method: Dosing was started after 3 days of stool mass removal using a glycerin enema, and if defecation was not observed for 4 days, a glycerin enema was used on the fifth day. During the course of administration, the defecation frequency, stool status scores (scores according to the Bristol Stool Scale), compliance, and side effects were recorded using a defecation diary.

Evaluation method: Defecation frequency and stool status scores were evaluated during the second and third weeks after the administration. Defecation frequency did not include those excreted using glycerin enemas. As the primary evaluation scores, patients who met the following criteria were defined as those who were successfully treated for constipation: the frequency of defecation was at least 3 times/week, the stool status score was from 2 to 7, and no symptoms selected from encopresis, abdominal pain, pain during defecation, and bleeding during defecation was observed. With these criteria, the percentage of patients treated successfully for constipation was evaluated.

Table 10 shows the patient background for the patients administered with magnesium oxide.

**[Table 10]**

| Table 10. Patient background for the magnesium oxide administration group | | |
|---|---|---|
| Number of patients (number of evaluated cases) | | 52 (49) |
| Sex | Male (cases) | 20 |
| | Female (cases) | 32 |
| Age | Median | 2 years 11 months old |
| | Range | 8 months to 9 years 2 months old |
| Average frequency of spontaneous defecation (times/week)* | | 3.9±2.87 |
| Stool status score per week according to the Bristol Stool Scale (median)* | | 2.7±1.09 |

| | | |
|---|---|---|
| * As an observation period, the defecation status before administration was investigated to check defecation frequency and stool status scores in the untreated condition. | | |

When administered continuously for 3 weeks, the average frequency of defecation during the second and third weeks after the administration was 5.5 ± 3.15.

When administered continuously for 3 weeks, the average stool status score (score according to the Bristol Stool Scale) during the second and third weeks after the administration was 4.90 ±0.91.

Patients with a compliance rate of at least 75% of the target period after 3 weeks of continuous administration were considered evaluable patients.

At more than one week after administration, the serum magnesium concentrations of the 32 patients examined ranged from 2.1 to 2.8 mg/dL. None of the patients exhibited hypermagnesemia.

Patients who met the following criteria were defined as those who were successfully treated for constipation: the frequency of defecation was at least 3 times/week, the stool status score was from 2 to 7, and no symptoms selected from encopresis, abdominal pain, pain during defecation, and bleeding during defecation was observed. With these criteria, the percentage of patients treated successfully for constipation was 41% (20 of 49 cases).

Since 20 of the 49 cases were successfully treated cases, the prior distribution has 20 treatment successes and 29 failures. The prior distribution and likelihood were combined to obtain a posterior distribution with 21 treatment successes and 30 failures. From this posterior distribution, the probability that the posterior distribution treatment success probability was 0.7 or less was calculated to be 0.9999894. That is, the probability that the treatment success rate was 70% or more was 0.001%.

### INDUSTRIAL APPLICABILITY

The present invention can be widely used in the treatment of pediatric constipation patients, for whom effective treatment methods have been limited, and therefore has an extremely high industrial values.

## Claims

1. A pharmaceutical composition comprising magnesium oxide as an active ingredient for treating constipation, which is administered orally to a pediatric patient with a body weight of 7.5kg or more and an age of from 1 to 5 years old twice daily such that a daily dosage of magnesium oxide is from 25 to 45 mg/kg.

2. The pharmaceutical composition according to claim 1, which is in the form of tablets or fine granules.

3. The pharmaceutical composition according to claim 1 or 2, wherein the twice daily administration includes administrations after breakfast and after dinner.

4. The pharmaceutical composition according to any one of claims 1 to 3, wherein the daily dosage is administered as 2n units, which are divided into n units after breakfast and n units after dinner.

5. The pharmaceutical composition according to any one of claims 1 to 3, wherein the daily dosage is administered as 2n+1 units, which are divided into n units after breakfast and n+1 units after dinner.

6. The pharmaceutical composition according to any one of claims 1 to 5, wherein the constipation is chronic functional constipation.
